(19) Europäisches Patentamt
European Patent Office
Office européen des brevets

(11) Veröffentlichungsnummer : **0 429 856 B1**

(12) # EUROPÄISCHE PATENTSCHRIFT

(45) Veröffentlichungstag der Patentschrift : **19.01.94 Patentblatt 94/03**

(51) Int. Cl.$^5$ : **A61K 31/19,** A61K 9/02, A61K 9/08

(21) Anmeldenummer : **90120443.8**

(22) Anmeldetag : **25.10.90**

(54) **Neue Arzneizubereitungen, ein Metallsalz des Ibuprofens enthaltend und Verfahren zur Herstellung derselben.**

Die Akte enthält technische Angaben, die nach dem Eingang der Anmeldung eingereicht wurden und die nicht in dieser Patentschrift enthalten sind.

(30) Priorität : **17.11.89 DE 3938227**
**18.07.90 DE 4022814**

(43) Veröffentlichungstag der Anmeldung : **05.06.91 Patentblatt 91/23**

(45) Bekanntmachung des Hinweises auf die Patenterteilung : **19.01.94 Patentblatt 94/03**

(84) Benannte Vertragsstaaten : **AT BE CH DE DK ES FR GB IT LI LU NL SE**

(56) Entgegenhaltungen :
**EP-A- 0 257 288**
**EP-A- 0 279 519**
**EP-A- 0 298 740**
**EP-A- 0 300 277**
**FR-A- 2 400 363**

(73) Patentinhaber : **Dolorgiet GmbH & Co. KG**
**Otto-von-Guericke-Str. 1**
**D-53757 Sankt Augustin (DE)**

(72) Erfinder : **Löhner, Manfred**
**Blücherstrasse 4**
**W-5300 Bonn 1 (DE)**
Erfinder : **Posselt, Klaus, Dr.**
**Rodderbergstrasse 62**
**W-5300 Bonn 2 (DE)**

(74) Vertreter : **Werner, Hans-Karsten, Dr. et al**
**Patentanwälte Von Kreisler-Selting-Werner**
**Postfach 10 22 41**
**D-50462 Köln (DE)**

EP 0 429 856 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue Arzneizubereitungen enthaltend ein Metallsalz des Ibuprofens, insbesondere in Form von Lösungen zum Einnehmen sowie Verfahren zu ihrer Herstellung.

Die Verbindung Ibuprofen, 2-(4-Isobutyl-phenyl)-propionsäure, ist z.B. aus Martindale, The Extra Pharmacopoeia, 29th edition, 1989, 20, als Arzneistoff bekannt, der antiinflammatorische und analgetische Eigenschaften besitzt. Er wird eingesetzt zur Behandlung von rheumatoider Arthritis oder anderen entzündlichen Gelenkerkrankungen, Weichteilrheumatismus und Gicht. Wegen seiner analgetischen Eigenschaften findet Ibuprofen auch eine breite Anwendung als Schmerzmittel.

Zum Einnehmen ist Ibuprofen überwiegend in festen Formen, als Dragées, Tabletten oder Kapseln im Handel. Aus den Patentanmeldungen EP 0 070 714 und EP 0 178 436 sind auch Weichgelatinekapseln bekannt, in denen Ibuprofen suspendiert bzw. gelöst vorliegt. Bekanntlich haben viele Patienten, z.B. Kinder und alte Personen, Schwierigkeiten solche Darreichungsformen zu schlucken. Für diese Patienten sind flüssige Arzneimittel und Suppositorien von Vorteil.

Aus den Patentanmeldungen DE 26 52 961 (entsprechend GB 1 527 563), DE 28 32 380 (entsprechend US 4 145 440) und DE 31 24 014 (entsprechend US 4 361 580) sind flüssige Arzneimittel bekannt, die Ibuprofen-Aluminiumsalz suspendiert enthalten. In den Patentanmeldungen DE 33 40 347 (entsprechend US 4 473 584, US 4 477 468 und US 4 701 470), EP 0 070 714 (entsprechend US 4 443 476 und US 4 447 451), EP 0 257 288 (entsprechend US 4 684 666), EP 0 264 187, GB 2 189 142 und US 4 346 108 werden Ibuprofen und seine pharmazeutisch akzeptablen Salze zur Anwendung in Arzneimitteln beschrieben.

In diesen Druckschriften sind jedoch nur Ausführungsbeispiele aufgeführt, in denen Ibuprofen-Aluminium, Ibuprofen-Natrium oder Ibuprofen als freie Säure verwendet werden. In der EP 0 137 668 werden neben der Anwendung von Flurbiprofen-Salzen auch Salze des Ibuprofens beansprucht, obwohl keine Beispiele für die Anwendung der Ibuprofensalze offenbart sind. Außerdem wird in der EP 0 298 740 (entsprechend US 4 788 220) eine flüssige pädiatrische Zubereitung beschrieben, in der Ibuprofen als freie Säure suspendiert vorliegt. Ferner ist aus der DE 31 05 357 das Zinksalz des Ibuprofens bekannt, welches in allen pharmazeutischen Zubereitungen verwendet werden kann, obwohl dafür keine Beispiele angegeben sind. Das Ibuprofen-Magnesiumsalz und Ibuprofen-Calciumsalz werden zwar in der DE 26 52 961 (entsprechend GB 1 527 563) beschrieben. Die beiden Salze werden bei einer Geschmacksprüfung als brennend eingestuft. Als Arzneimittel finden sie keine Anwendung. In der EP 0 279 519 werden pharmazeutische Zubereitungen beschrieben, die Ibuprofen-Salze in einem hydrophilen Träger enthalten. Bei allen Ausführungsbeispielen, einschließlich Suppositorien, wird aber ausschließlich das Natriumsalz eingesetzt.

Die EP-A-300 277 beschreibt 0,1 -5%ige Lösungen von Ibuprofen oder dessen Salzen in Wasser und/oder einem Benetzungsmittel wie zum Beispiel Glycerin und Sorbitolsirup.

Fast allen aus dem Stand der Technik bekannten flüssigen Zubereitungen und Suppositorien ist gemeinsam, daß der Wirkstoff Ibuprofen bzw. das Ibuprofensalz zumindest teilweise suspendiert vorliegt. Bekanntlich haben Suspensionen den Nachteil, daß bei der Herstellung nur unter großem Aufwand eine ausreichende Homogenität erzielt wird, und daß beim Lagern Phasentrennung erfolgen kann. Damit ist bei derartigen Arzneimitteln, die länger aufbewahrt wurden und nicht vorschriftsmäßig angewendet werden, eine exakte Dosierung nicht immer gewährleistet.

Die vorliegende Erfindung hat sich deshalb die Aufgabe gestellt, Ibuprofen bzw. seine Salze in physiologisch verträglichen Lösungsmitteln oder Lösungsmittelgemischen zu lösen und daraus Arzneimittelzubereitungen herzustellen, insbesondere in Form von Lösungen zum Einnehmen. Diese sollen einfach herzustellen und problemlos anzuwenden sein und eine gleichmäßig gute Verfügbarkeit des Wirkstoffes gewährleisten.

Diese Aufgabe kann überraschend einfach dadurch gelöst werden, daß die Arzneizubereitungen 0,9 bis 3,7 Gewichtsteile Ibuprofen-Magnesiumsalz gelöst in 5 bis 20 Gewichtsteilen Wasser, 0 bis 20 Gewichtsteilen 1,2 Propandiol, 30 bis 80 Gewichtsteilen Glycerin zusammen mit bis zu 2 Gewichtsteilen Süßstoff, bis 40 Gewichtsteilen Sorbitol als 70%ige wäßrige Lösung und/oder bis 25 Gewichtsteile Saccharose, wobei die Summe der Bestandteile 100 Gewichtsteile nicht überschreitet, enthalten.

Erfindungsgemäß können somit als alleinige Lösungsmittel weder das stark hydrophile Wasser noch das stark lipophile Paraffin verwendet werden. Ein gutes Lösungsmittel für das Ibuprofen-Magnesiumsalz ist Ethanol, welches sowohl hydrophile als auch lipophile Eigenschaften besitzt. Rein alkoholische Lösungen des Ibuprofen-Magnesiumsalzes sind jedoch als Arzneimittelzubereitungen weniger geeignet. Geeigneter sind hingegen bereits wäßrig alkoholische Lösungen, die obendrein 1,2-Propandiol enthalten. Weiterhin sind wäßrige Lösungen geeignet, die 1,2-Propandiol und niedermolekulares Polyethylenglykol enthalten. Als Zusatz zur wäßrigen Lösung ist auch Glycerin geeignet. Von der lipophilen Seite her kommend sind Neutralöle, wie Triglyceride, auch noch nicht sonderlich gut geeignet. Glycerinmonoricinoleat hingegen ist ein ausgezeichnetes Losungsmittel für Ibuprofenmagnesiumsalz. Weiterhin sehr gut geeignete Lösungsmittel sind polyoxyethylierte

Oleinglyceride und Polyoxyethylenglycerin-Kokosnußölfettsäureester sowie Isopropylmyristat. Um festzustellen, ob ein Lösungsmittel oder ein Lösungsmittelgemisch erfindungsgemäß verwendet werden kann, ist es somit sinnvoll, zuvor die Löslichkeit des Ibuprofen-Magnesiumsalzes hierin zu messen.

Zur Bestimmung der Löslichkeit der Ibuprofen-Salze werden bei Raumtemperatur gesättigte Lösungen hergestellt. Ein aliquoter Teil dieser Lösungen wird mit einem mit der Lösung mischbaren Lösungsmittel verdünnt und der Gehalt an Ibuprofen mittels HPLC über eine Reversed-Phase-Säule und UV-Detektion bestimmt. Die Löslichkeiten der Ibuprofen-Salze in einigen typischen Lösungsmitteln und Lösungsmittelgemischen sind in der Tabelle 1 zusammengestellt.

Das Ibuprofen-Magnesiumsalz hat die folgende Formel

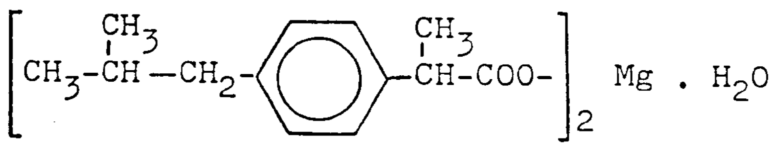

Vorzugsweise wird es folgendermaßen hergestellt: man suspendiert Ibuprofen in Wasser und gibt langsam unter Rühren die äquimolare Menge eines wasserlöslichen tertiären Amins (Triethylamin, Triethanolamin, Triisopropanolamin) zu, wobei sich das Ibuprofen auflöst. Aus dieser Lösung fällt man das Ibuprofen-Magnesiumsalz durch Zugabe einer wäßrigen Lösung der äquimolaren Menge Magnesiumchlorid. Nach 12-stündigem Stehen wird das Salz abgesaugt, mit kaltem Wasser aminfrei gewaschen und bei 45°C getrocknet. Das Ibuprofen-Magnesiumsalz enthält 5,4 % Magnesium und 3,9 % Wasser entsprechend der oben angegebenen Formel und schmilzt bei 165 - 170°C.

Bevorzugte erfindungsgemäße flüssige Zubereitungen, wie Saft oder Sirup enthalten vorteilhaft bis 5 Gewichtsteile Ethanol.

In einer besonderen Ausführungsform sind zur Geschmacksverbesserung bis zu 2 Gewichtsteile Geschmackskorrigenzien und/oder Aromastoffe enthalten. Darüber hinaus kann es von Vorteil sein, ein Netzmittel (bis zu 0,2 Gewichtsteile Polyvinylpyrrolidin) zur Beschleunigung des Lösungsvorgangs zuzuführen.

Eine weitere Ausführungsform, die speziell der Bekämpfung starker Schmerzen dient, enthält zusätzlich 0,07 bis 0,2 Gewichtsteile Codein, vorzugsweise in Form eines Salzes, wie Codeinhydrochlorid oder Codeinsulfat.

Die Lösungen werden vorzugsweise dadurch hergestellt, daß man die festen Stoffe zunächst in 1,2-Propandiol suspendiert, die Aromen zufügt und nach Zugabe des Wassers und gegebenenfalls Ethanols solange bei Raumtemperatur oder leicht erhöhter Temperatur (bis 40°C) rührt, bis eine klare Lösung entstanden ist. Dann fügt man nacheinander bei Raumtemperatur unter Rühren das Glycerin und die wäßrige Sorbitol- und/oder Saccharoselösung zu, wobei zwar eine vorübergehende Trübung auftreten kann, die jedoch wieder verschwindet.

Die Lösungen können auch dadurch hergestellt werden, daß man die Süßstoffe und gegebenenfalls Netzmittel und/oder Codein bzw. Codeinsalz in Wasser löst, Glycerin und die Lösung der Aromen und gegebenenfalls Geschmackskorrigenzien in 1,2-Propandiol bzw. Ethanol und einem Teil des Glycerins zufügt und dann das Ibuprofen-Magnesiumsalz portionsweise bei Raumtemperatur bis zur vollständigen Lösung einrührt.

Die Freisetzung des Ibuprofens aus erfindungsgemäßen Lösungen wurde nach dem Modell der USP XXI mittels der Paddle-Methode in 900 ml künstlichem Magensaft bei 37°C und 100 RPM geprüft. Die Konzentration des Ibuprofens wurde UV-spektrometrisch bei 231 nm bestimmt. Zum Vergleich wurden Zubereitungen aus dem o.g. Stand der Technik herangezogen. Die Messungen wurden jeweils mit äquimolaren Mengen des Salzes entsprechend 200 mg Ibuprofen durchgeführt. In Tabelle 2 sind die Werte des freigesetzten Ibuprofens aus der erfindungsgemäßen Zubereitung und den Vergleichspräparaten zusammengestellt. Es wurden auch mittels der Trapezregel die Flächen unter den Wirkstoffmengen-ZeitKurven (AUC) berechnet. Diese Flächenwerte stellen ein Maß für die in der Meßzeit freigesetzten Mengen an Ibuprofen dar. Aus den Einzelwerten ist zu erkennen, daß die erfindungsgemäßen Lösungen Ibuprofen wesentlich schneller freisetzten als die Vergleichspräparate, die auch innerhalb der ersten 20 Minuten relativ weniger Ibuprofen (52 - 100 Teile) freisetzen als die erfindungsgemäßen Lösungen (108 - 131 Teile).

Zusammensetzung der Vergleichspräparate:

Präparat A (entsprechend DE 26 52 961 und GB 1 527 563

| | |
|---|---|
| Aluminiumibuprofen (1 : 1) | 2,6 g |
| Mikrokristalline Cellulose | 1,2 g |

| | |
|---|---|
| Natriumcarboxymethylcellulose | 1,0 g |
| Wasser | ad 100,0 ml |

Präparat B (entsprechend DE 28 32 380 und US 4 145 440)

| | |
|---|---|
| Aluminiumdiibuprofen (1 : 2) | 2,2 g |
| Avicel[R] RC-591 | 1,5 g |
| Polysorbat | 0,5 g |
| Glycerin | 10,0 ml |
| Sorbitol 70 % | 20,0 ml |
| Wasser | ad 100,0 ml |

Präparat C (entsprechend DE 31 24 014 und US 4 361 580)

| | |
|---|---|
| Aluminiumdiibuprofen (1 : 2) | 2,2 g |
| Avicel[R] RC-591 | 1,0 g |
| Polysorbat 80 | 0,5 g |
| Saccharose | 50,0 g |
| Wasser | ad 100,0 ml |

Präparat D (entsprechend DE 33 40 347, EP 0 070 714, US 4 346 108, US 4 443 476, US 4 447 451, US 4 473 584, US 4 477 468 und US 4 701 470

| | |
|---|---|
| Aluminiumibuprofen (1 : 1) | 2,6 g |
| Zitronensäure | 0,2 g |
| Saccharose | 70,0 g |
| Traganth | 0,5 g |
| Wasser | ad 100,0 ml |

Präparat E (entsprechend wie Präparat D)

| | |
|---|---|
| Aluminiumdiibuprofen (1 : 2) | 2,2 g |
| Zitronensäure | 0,2 g |
| Saccharose | 70,0 g |
| Traganth | 0,5 g |
| Wasser | ad 100,0 ml |

Präparat F (entsprechend wie Präparat D)

| | |
|---|---|
| Ibuprofencalcium (2 : 1) | 2,2 g |
| Zitronensäure | 0,2 g |
| Saccharose | 70,0 g |
| Traganth | 0,5 g |
| Wasser | ad 100,0 ml |

Präparat G (entsprechend EP 0 257 288 und US 4 684 666)

| | |
|---|---|
| Ibuprofen | 2,0 g |
| Sorbitol | 61,3 g |
| Glycerin | 18,8 g |
| Ethanol | 21,1 g |
| Veegum® | 0,26 g |
| 1,2-Propandiol | 0,7 g |
| PVP | 0,13 g |
| Natriumhydroxid | 0,55 g |
| Wasser | ad 100,0 ml |

Präparat H (entsprechend wie Präparat G)

| | |
|---|---|
| Aluminiumdiibuprofen (1 : 2) | 2,2 g |
| Sorbitol | 42,3 g |
| 1,2-Propandiol | 21,8 g |
| Ethanol | 21,1 g |
| Veegum | 0,26 g |
| PVP | 0,13 g |
| Saccharose | 29,8 g |
| Kaliumhydroxid | 0,63 g |
| Wasser | ad 100,0 ml |

Präparat J (entsprechend EP 0 264 187)

| | |
|---|---|
| Ibuprofen | 2,0 g |
| Sorbitol | 10,0 g |
| Glycerin | 15,0 g |
| Mikrokristalline Cellulose | 2,5 g |
| Aluminiumhydroxid Gel BP | 2,0 g |
| Wasser | ad 100,0 ml |

Präparat K (entsprechend GB 2 189 142)

| | |
|---|---|
| Ibuprofen | 2,0 g |
| Saccharose | 66,0 g |
| Zitronensäure | 0,25 g |
| Agar BPC | 0,30 g |
| Glycerin | 10,0 g |
| Sorbitol Lösung BPC | 10,0 g |
| Kaolin BP | 1,0 g |
| Polysorbat 80 | 0,10 g |
| Wasser | ad 100,0 ml |

Präparat L (entsprechend EP 0 298 740 und US 4 788 220)

| | |
|---|---|
| Ibuprofen | 2,0 g |
| Xanthan Gummi | 0,1 g |
| Mikrokristalline Cellulose | 0,75 g |
| Zitronensäure | 0,25 g |
| Saccharose | 50,0 g |
| Glycerin | 10,0 g |
| Sorbitol Lösung USP | 10,0 g |
| Natriumcarboxymethylcellulose | 0,1 g |
| Polysorbat 80 | 0,10 g |
| Wasser | ad 100,0 ml |

Präparat M (Salz entsprechend DE 31 05 357; Zusammensetzung erfindungsgemäß)

| | |
|---|---|
| Ibuprofenzink (2 : 1) | 2,47 g |
| β-Cyclodextrin | 1,4 g |
| Calciumcyclamat | 1,15 g |
| 1,2-Propandiol | 17,6 g |
| Wasser | 10,0 g |
| Glycerin | 40,0 g |
| Sorbitol Lösung BP | ad 100,0 ml |

Die nachstehenden Beispiele erläutern die Erfindung:

Beispiel 1

Man suspendiert 21,96 g Ibuprofen-Magnesiumsalz und 20,0 g Calciumcyclamat in 176,0 g 1,2-Propandiol, gibt 6,0 g Schwarzkirscharoma und 100,0 g Wasser zu und rührt bei Raumtemperatur so lange bis eine klare Lösung entstanden ist. Dann rührt man 420,0 g Glycerin ein und füllt unter Rühren bei Raumtemperatur mit (ca. 461 g) Sorbitol-Lösung 70 % kristallisierend (DAB 9) auf 1000 ml (ca. 1206 g) auf.

5 ml (1 Teelöffel) der Lösung enthalten 109,8 mg (Ibuprofen-Magnesiumsalz entsprechend 100,0 mg Ibuprofen.

Beispiel 2

Analog Beispiel 1 wird eine Lösung folgender Zusammensetzung hergestellt:
21,96 g Ibuprofen-Magnesiumsalz
20,00 g Calciumcyclamat
176,00 g 1,2-Propandiol
6,00 g Schwarzkirscharoma
80,00 g Wasser
400,00 g Glycerin
250,00 g Saccharose-Wasser-Lösung (1:1;w/w)
(ca. 236 g) Sorbitol-Lösung 70 % kristallisierend (DAB 9) ad 1000 ml (ca. 1192 g).
5 ml (1 Teelöffel) der Lösung enthalten 109,8 mg Ibuprofen-Magnesiumsalz entsprechend 100,0 mg Ibuprofen.

Beispiel 3

Analog Beispiel 1 wird eine Lösung folgender Zusammensetzung hergestellt:
21,96 g Ibuprofen-Magnesiumsalz
20,00 g Calciumcyclamat
176,00 g 1,2-Propandiol
11,00 g Ethanol
6,00 g Schwarzkirscharoma
80,00 g Wasser
400,00 g Glycerin
250,00 g Saccharose-Wasser-Lösung (1:1;w/w)
(ca. 205 g) Sorbitol-Lösung 70 % kristallisierend (DAB 9) ad 1000 ml (ca. 1171 g).

5 ml (1 Teelöffel) der Lösung enthalten 109,8 mg Ibuprofen-Magnesiumsalz entsprechend 100,0 mg Ibuprofen.

Beispiel 4

Man löst 21,96 g Ibuprofen-Magnesiumsalz und 0,20 g Menthol in 31,6 g Ethanol und fügt 151,2 g Glycerin und 2,0 g Schwarzkirscharoma zu. Diese Lösung rührt man bei Raumtemperatur in eine Lösung von 1,4 g Polyvinylpyrrolidon (Kollidon$^R$ 25), 10,0 g Natriumcyclamat, 200,0 g Saccharose und 120,0 g Sorbitol-Lösung 70% kristallisierend (DAB 9) in 200,0 g Wasser und 22,1 g Ethanol ein und füllt die Mischung mit Glycerin (ca. 76 g) auf ein Volumen von 1000,0 ml (ca. 1173 g) auf.

5 ml (1 Teelöffel) der Lösung enthalten 109,8 mg Ibuprofen-Magnesiumsalz entsprechend 100,0 mg Ibuprofen.

Beispiel 5

Man löst 1,4 g Polyvinylpyrrolidon, 15,0 g Natriumcyclamat und 1,5 g Saccharin-Natrium in 200,0 g Wasser, fügt 504,4 g Glycerin und eine Lösung von 0,20 g Menthol und 3,0 g Bitterschokoladenaroma in 10,4 g 1,2-Propandiol und 126,1 g Glycerin zu. In diese Mischung rührt man bei Raumtemperatur 21,96 g Ibuprofen-Magnesiumsalz ein. Nach vollständiger Lösung füllt man mit Glycerin (ca. 76 g) auf 1000,0 ml (ca. 1243 g) auf.

5 ml (1 Teelöffel) der Lösung enthalten 109,8 mg Ibuprofen-Magnesiumsalz entsprechend 100,0 mg Ibuprofen.

Beispiel 6

Man löst 15,0 g Natriumcyclamat und 1,5 g Saccharin-Natrium in 200,0 g Wasser, fügt 504,4 g Glycerin und eine Lösung von 0,20 g Menthol und 3,0 g Bitterschokoladenaroma in 10,4 g 1,2-Propandiol und 126,1 g Glycerin zu. In diese Mischung rührt man bei Raumtemperatur 21,96 g Ibuprofen-Magnesiumsalz ein. Nach vollständiger Lösung füllt man mit Glycerin (ca. 305 g) auf 1000,0 ml (ca. 1187 g) auf.

5 ml (1 Teelöffel) der Lösung enthalten 109,8 mg Ibuprofen-Magnesiumsalz entsprechend 100,0 mg Ibuprofen.

Beispiel 7

Analog Beispiel 6 stellt man eine Lösung folgender Zusammensetzung her:
10,98 g Ibuprofen-Magnesiumsalz
10,00 g Natriumcyclamat
1,00 g Saccharin-Natrium
200,00 g Wasser
504,40 g Glycerin
0,20 g Menthol
2,00 g Bitterschokoladenaroma
10,40 g 1,2-Propandiol
126,10 g Glycerin
und füllt die Lösung mit Glycerin (ca. 83 g) auf 1000,0 ml (ca. 1190 g) auf.

5 ml (1 Teelöffel) der Lösung enthalten 54,9 mg Ibuprofen-Magnesiumsalz entsprechend 50,0 mg Ibuprofen.

Beispiel 8

Analog Beispiel 6 stellt man eine Lösung folgender Zusammensetzung her:
43,92 g Ibuprofen-Magnesiumsalz
15,00 g Natriumcyclamat
1,50 g Saccharin Natrium
504,40 g Glycerin
200,00 g Wasser
0,20 g Menthol
3,00 g Bitterschokoladenaroma
10,40 g 1,2-Propandiol
126,10 g Glycerin
und füllt die Lösung mit Glycerin (ca. 75 g) auf 1000,0 ml (ca. 1198 g) auf.

5 ml (1 Teelöffel) der Lösung enthalten 219,6 mg Ibuprofen-Magnesiumsalz entsprechend 200,0 mg Ibuprofen.

Beispiel 9

Man löst 15,0 g Natriumcyclamat, 1,5 g Saccharin-Natrium und 1,144 g Codein-Hydrochlorid-Dihydrat in 200,0 g Wasser, fügt 506,0 g Glycerin und eine Lösung von 0,20 g Menthol und 3,0 g Bitterschokoladenaroma in 10,4 g 1,2-Propandiol und 120,0 g Glycerin zu. In diese Mischung rührt man bei Raumtemperatur 21,96 g Ibuprofen-Magnesiumsalz ein. Nach vollständiger Lösung füllt man mit Glycerin (ca. 309 g) auf 1000,0 ml (ca. 1189 g) auf.

5 ml (1 Teelöffel) der Lösung enthalten 109,8 mg Ibuprofen-Magnesiumsalz entsprechend 100,0 mg Ibuprofen und 5,72 mg Codein-Hydrochlorid-Dihydrat entsprechend 4,60 mg Codein.

Tabelle 1: Löslichkeit von Ibuprofen-Salzen bei Raumtemperatur (mg Ibuprofen/ml)

| Lösungsmittel | Mg | Ca | Ibuprofen-Al (2 : 1) | Zn | Na |
|---|---|---|---|---|---|
| Wasser | 7 | 3 | < 0,1 | 0,4 | 200 |
| Ethanol | 500 | 100 | 2 | 250 | 150 |
| 1,2-Propandiol | 160 | 330 | 0,8 | 13 | 500 |
| Glycerin | 8 | 20 | < 0,1 | 0,7 | 330 |
| Polyethylenglykol 400 | 40 | 38 | 2 | 47 | 250 |
| Ethanol/1,2-Propandiol/Wasser (1/4/4;v/v/v) | 330 | 12 | < 0,1 | 1 | 500 |
| 1,2-Propandiol/Polyethylenglykol 400/Wasser (5/3/6;v/v/v) | 125 | 3 | < 0,1 | 0,8 | 200 |
| 1,2-Propandiol/Glycerin/Wasser (2/4/3;v/v/v) | 250 | 7 | < 0,1 | 0,7 | 330 |
| Isopropylmyristat | 260 | 0,3 | 1 | 6 | 0,3 |
| Caprylic/Capric Triglyceride (Miglyol 812) | 17 | 0,2 | 0,3 | 7 | < 0,1 |
| Polyoxyethylierte Olein-Glyceride (Labrafil M 1944 CS) | 380 | 37 | 4 | 50 | 38 |
| Cabrylic/Capric Triglyceride (Miglyol 810) | 35 | 0,4 | 0,5 | 5 | 0,2 |
| Glycerinmonoricinoleat | 450 | 64 | 4,5 | 133 | 95 |
| Paraffinum supl. | 0,4 | < 0,1 | < 0,1 | < 0,1 | < 0,1 |
| Polyoxyethylen-(7)-glycerin-kokosnußölfett-säure-ester | 480 | 10 | 6 | 8 | 120 |

Tabelle 2: Freisetzung von Ibuprofen

| | mg Ibuprofen nach Minuten | | | | | | | | | | AUC | |
|---|---|---|---|---|---|---|---|---|---|---|---|---|
| | 2 | 4 | 6 | 8 | 10 | 12 | 14 | 16 | 18 | 20 | mg x min. | relativ |
| Präp. A | 18,6 | 32,6 | 37,6 | 42,0 | 45,0 | 47,2 | 49,4 | 51,8 | 53,4 | 55,6 | 810 | 87 |
| Präp. B | 15,2 | 19,6 | 26,6 | 29,6 | 32,4 | 37,2 | 41,2 | 44,6 | 47,0 | 50,2 | 637 | 68 |
| Präp. C | 9,2 | 22,0 | 30,0 | 36,4 | 41,4 | 46,4 | 50,6 | 54,6 | 57,8 | 61,0 | 757 | 81 |
| Präp. D | 8,2 | 22,8 | 26,6 | 30,6 | 32,8 | 34,0 | 36,6 | 38,0 | 39,0 | 40,6 | 577 | 62 |
| Präp. E | 12,8 | 33,4 | 42,0 | 47,6 | 51,8 | 55,6 | 58,8 | 61,4 | 63,8 | 66,0 | 920 | 99 |
| Präp. F | 5,6 | 26,0 | 29,2 | 31,0 | 32,2 | 33,0 | 33,6 | 34,0 | 34,4 | 34,8 | 552 | 59 |
| Präp. G | 30,8 | 60,2 | 64,1 | 62,4 | 54,3 | 48,0 | 44,2 | 41,7 | 40,6 | 39,7 | 932 | 100 |
| Präp. H | 6,8 | 46,6 | 54,7 | 56,0 | 52,8 | 48,8 | 45,8 | 43,8 | 41,5 | 41,0 | 834 | 89 |
| Präp. J | 0,8 | 9,6 | 17,1 | 22,5 | 27,0 | 31,6 | 34,8 | 38,1 | 40,7 | 42,4 | 486 | 52 |
| Präp. K | 5,0 | 30,7 | 37,2 | 39,7 | 41,0 | 41,8 | 42,2 | 43,0 | 43,4 | 43,5 | 691 | 74 |
| Präp. L | 4,8 | 32,1 | 35,7 | 37,6 | 38,4 | 39,5 | 40,0 | 40,4 | 41,0 | 41,4 | 660 | 71 |
| Präp. M | 12,5 | 24,8 | 27,8 | 29,1 | 30,2 | 30,8 | 31,6 | 32,0 | 32,6 | 33,2 | 536 | 57 |
| Beisp. 3 | 28,3 | 67,9 | 75,8 | 75,4 | 72,9 | 69,0 | 65,8 | 63,4 | 61,6 | 60,2 | 1220 | 131 |
| Beisp. 5 | 20,3 | 69,4 | 77,5 | 72,2 | 62,7 | 53,2 | 46,0 | 41,4 | 39,1 | 38,0 | 1002 | 108 |
| Beisp. 6 | 19,4 | 61,9 | 69,6 | 67,7 | 62,4 | 59,2 | 56,5 | 53,6 | 50,5 | 48,0 | 1050 | 113 |

## Patentansprüche

1. Arzneizubereitungen enthaltend ein Metallsalz des Ibuprofens zur oralen Einnahme, dadurch gekennzeichnet, daß sie enthalten 0,9 bis 3,7 Gewichtsteile Ibuprofen-Magnesiumsalz gelöst in 5 bis 20 Gewichtsteilen Wasser, 0 bis 20 Gewichtsteilen 1,2-Propandiol, 30 bis 80 Gewichtsteilen Glycerin zusammen mit bis zu 2 Gewichtsteilen Süßstoff, bis zu 40 Gewichtsteilen Sorbitol als 70 %ige wäßrige Lösung und/ oder bis 25 Gewichtsteilen Saccharose, wobei die Summe der Bestandteile 100 Gewichtsteile nicht überschreitet.

2. Arzneizubereitungen zur oralen Einnahme gemäß Anspruch 1, dadurch gekennzeichnet, daß sie zur Geschmacksverbesserung bis zu 2 Gewichtsteile Geschmackskorrigenzien und/oder Aromastoffe enthalten.

3. Arzneizubereitungen zur oralen Einnahme gemäß Anspruch 1 oder 2, dadurch gekennzeichnet, daß sie zusätzlich bis zu 5 Gewichtsteile Ethanol enthalten.

4. Arzneizubereitungen zur oralen Einnahme gemäß Anspruch 1, 2 oder 3, dadurch gekennzeichnet, daß sie zusätzlich bis zu 0,2 Gewichtsteile Netzmittel enthalten.

5. Arzneizubereitungen zur oralen Einnahme gemäß Anspruch 1 bis 4, dadurch gekennzeichnet, daß sie zusätzlich 0,07 bis 0,2 Gewichtsteile Codein oder Codeinsalz enthalten.

6. Verfahren zur Herstellung von Arzneizubereitungen enthaltend ein Metallsalz des Ibuprofens zur oralen Einnahme, dadurch gekennzeichnet, daß
a) 0,9 bis 3,7 Gewichtsteile Ibuprofen-Magnesiumsalz und bis zu 2 Gewichtsteile Süßstoff in 0 bis 20 Gewichtsteile 1,2-Propandiol suspendiert, bis zu 2 Gewichtsteile Aromastoffe und 5 bis 20 Gewichtsteile Wasser zugefügt werden und bei Temperaturen bis zu 40°C bis zur vollständigen Lösung gerührt wird, bei Raumtemperatur unter Rühren 30 bis 80 Gewichtsteile Glycerin zugefügt werden und schließlich mit 50 %iger wäßriger Saccharose-Lösung und/oder 70 %iger wäßriger Sorbitol-Lösung auf 100 Volumenteile aufgefüllt wird oder daß
b) bis zu 2 Gewichtsteile Süßstoff in 5 bis 20 Gewichtsteilen Wasser gelöst werden, bis 45 Gewichtsteile Glycerin und eine Lösung von bis zu 2 Gewichtsteilen Geschmackskorrigenz und/oder Aromastoff in bis zu 1 Gewichtsteil 1,2-Propandiol und bis zu 12 Gewichtsteilen Glycerin zugefügt werden, 0,9 bis 3,7 Gewichtsteile Ibuprofen-Magnesiumsalz portionsweise bis zur vollständigen Lösung eingerührt werden und mit Glycerin auf 100 Volumenteile aufgefüllt wird.

7. Verfahren gemäß Anspruch 6, dadurch gekennzeichnet, daß nach der Suspendierung der festen Stoffe in 1,2-Propandiol und Zugabe der Aromen zum Lösen neben Wasser zusätzlich bis zu 5 Gewichtsteile Ethanol verwendet werden.

8. Verfahren nach Anspruch 7, dadurch gekennzeichnet, daß in Variante b) zusätzlich bis zu 0,2 Gewichtsteile Netzmittel im Wasser gelöst werden.

9. Verfahren nach Anspruch 7 oder 8, dadurch gekennzeichnet, daß in Variante b) zusätzlich 0,07 bis 0,2 Gewichtsteile Codein oder Codeinsalz im Wasser gelöst werden.

## Claims

1. Medicament formulations for oral intake containing a metal salt of ibuprofen, characterized in that they contain from 0.9 to 3.7 parts by weight of ibuprofen-magnesium salt dissolved in from 5 to 20 parts by weight of water, from 0 to 20 parts by weight of 1,2-propanediol and from 30 to 80 parts by weight glycerol together with up to 2 parts by weight of a sweetener, up to 40 parts by weight of sorbitol as a 70% aqueous solution and/or up to 25 parts by weight of saccharose, with the proviso that the sum of the components does not exceed 100 parts by weight.

2. The medicament formulations for oral intake according to claim 1, characterized in that they contain up to 2 parts by weight of flavour correctives and/or aroma substances for improving the flavour thereof.

3. The medicament formulations for oral intake according to claims 1 or 2, characterized in that they additionally contain up to 5 parts by weight of ethanol.

4. The medicament formulations for oral intake according to claims 1, 2 or 3, characterized in that they additionally contain up to 0.2 parts by weight of a wetting agent.

5. The medicament formulations for oral intake according to claims 1 to 4, characterized in that they additionally contain from 0.07 to 0.2 parts by weight of codeine or codeine salt.

6. A process for preparing medicament formulations for oral intake containing a metal salt of ibuprofen, characterized in that
   a) from 0.9 to 3.7 parts by weight of ibuprofen-magnesium salt and up to 2 parts by weight of sweeteners are suspended in from 0 to 20 parts by weight of 1,2-propanediol, that up to 2 parts by weight of an aroma substance and from 5 to 20 parts by weight of water are added thereto and the mixture is stirred at 40 °C until complete dissolution has occurred, and that from 30 to 80 parts by weight glycerol are added thereto with stirring at room temperature, and eventually the resulting product mixture is filled up with a 50% aqueous saccharose solution and/or a 70% aqueous solution of sorbitol to make 100 parts by volume, or that
   b) up to 2 parts by weight of sweeteners are dissolved in from 5 to 20 parts by weight of water, up to 45 parts by weight of glycerol and a solution of up to 2 parts by weight of a flavour-corrective and/or aroma substance in up to 1 part by weight of 1,2-propanediol and up to 12 parts by weight of glycerol is added thereto, from 0.9 to 3.7 parts by weight of ibuprofen-magnesium salt are portionwise added while stirring the mixture until complete dissolution has occurred, and the resulting product mixture is filled up with glycerol to make 100 parts by volume.

7. The process according to claim 6, characterized in that, after suspending the solids in 1,2-propanediol and addition of the aromatizing ingredients, up to 5 parts by weight of ethanol are used for dissolving in addition to water.

8. The process according to claim 7, characterized in that in variant b) up to 0.2 parts by weight of a wetting agent are additionally dissolved in the water.

9. The process according to claims 7 or 8, characterized in that in variant b) from 0.07 to 0.2 parts by weight of codeine or codeine salt are dissolved in the water.

**Revendications**

1. Préparations médicales contenant un sel métallique de l'ibuprofène, pour administration orale, caractérisées en ce qu'elles contiennent 0,9 à 3,7 parties en poids d'un sel de magnésium de l'ibuprofène dissous dans 5 à 20 parties en poids d'eau, 0 à 20 parties en poids de 1,2-propane-diol, 30 à 80 parties en poids de glycérine, en association avec jusqu'à 2 parties en poids de saccharine, jusqu'à 40 parties en poids de sorbitol en solution aqueuse à 70 % et/ou jusqu'à 25 parties en poids de saccharose, la somme des parties des composants ne dépassant pas 100 parties en poids.

2. Préparations selon la revendication 1,
   caractérisées en ce qu'elles contiennent, pour améliorer le goût, jusqu'à 2 parties en poids de correcteurs de saveur et/ou de substances aromatiques.

3. Préparations selon la revendication 1,
   caractérisées en ce qu'elles contiennent en plus jusqu'à 5 parties en poids d'éthanol.

4. Préparations selon l'une des revendications 1 à 3,
   caractérisées en ce qu'elles contiennent en plus jusqu'à 0,2 parties en poids d'agent mouillant.

5. Préparations selon l'une des revendications 1 à 4,
   caractérisées en ce qu'elles contiennent en plus 0,07 à 0,2 parties en poids de codéine ou d'un sel de codéine.

6. Procédé d'obtention de préparations médicales contenant un sel métallique de l'ibuprofène, pour administration orale,
   caractérisé en ce que :
   a) on met en suspension 0,9 à 3,7 parties en poids d'un sel de magnésium de l'ibuprofène et jusqu'à

2 parties en poids de saccharine dans 0 à 20 parties en poids de 1,2-propane-diol, on ajoute jusqu'à 2 parties en poids de substances aromatiques et 5 à 20 parties en poids d'eau, et on agite à des températures jusqu'à 40°C jusqu'à dissolution complète, on ajoute 30 à 80 parties en poids de glycérine, sous agitation et à température ordinaire, et on complète enfin jusqu'à 100 parties en volume avec une solution aqueuse de saccharose à 50 % et/ou une solution aqueuse de sorbitol à 70 %, ou en ce que : b) on dissout jusqu'à 2 parties en poids de saccharine dans 5 à 20 parties en poids d'eau, on ajoute jusqu'à 45 parties en poids de glycérine et une solution de jusqu'à 2 parties en poids de correcteur de goût et/ou de substance aromatique dans jusqu'à 1 partie en poids de 1,2-propane-diol et jusqu'à 12 parties en poids de glycérine, on mélange avec agitation 0,9 à 3,7 parties en poids de sel de magnésium de l'ibuprofène jusqu'à dissolution complète, et on complète jusqu'à 100 parties en volume avec de la glycérine.

7. Procédé selon la revendication 5, caractérisé en ce qu'après la mise en suspension des substances solides dans le 1,2-propane-diol et l'addition des substances aromatiques, on utilise pour les dissoudre, en plus de l'eau, jusqu'à 5 parties en poids d'éthanol.

8. Procédé selon la revendication 7, caractérisé en ce que, dans la variante b), on dissout en plus dans l'eau, jusqu'à 0,2 parties en poids d'agent mouillant.

9. Procédé selon la revendication 7 ou 8, caractérisé en ce que dans la variante b), on dissout en plus dans l'eau 0,07 à 0,2 parties en poids de codéine ou d'un sel de codéine.